# EUROPÄISCHE PATENTANMELDUNG

(11) **EP 0 695 739 A1**
(43) Veröffentlichungstag der Anmeldung: **07.02.1996**
(21) Anmeldenummer: 95111425.5
(22) Anmeldetag: 20.07.1995
(51) Int. Cl.: C07C 231/08, C07C 233/15

(54) **Verfahren zur Herstellung nitrosubstituierter Arylamide und Arylamine**

(30) Priorität: 02.08.1994 DE 4427248
(71) Anmelder: BAYER AG, D-51368 Leverkusen (DE)
(72) Erfinder: Jautelat, Manfred, Dr., D-51399 Burscheid (DE); Arlt, Dieter, Prof. Dr., D-51065 Köln (DE); Makosza, Mieczystaw, Prof. Dr., PL-01-496 Warszawa (PL)

(57) **Zusammenfassung**

Nitroaromaten können mit Amiden in Gegenwart von Basen und Sauerstoff amidiert werden; durch Hydrolyse der entstandenen Amide sind die entsprechenden Amine zugänglich.

## Beschreibung

Die Erfindung betrifft ein Verfahren zur Herstellung von nitrosubstituierten Arylamiden und nitrosubstituierten Arylaminen aus Nitroaromaten, Säureamiden und Sauerstoff in Gegenwart von Basen.

Aromatische Amine sind wichtige Zwischenprodukte für die Herstellung von Farbstoffen, Pflanzenschutzmitteln, Pharmazeutika und für die Fotoindustrie.

Technisch bedeutsame Verfahren zur Herstellung von aromatischen Aminen sind die Reduktion der Nitrogruppen von leicht zugänglichen Nitroaromaten und die Umsetzung von Halogenaromaten mit Ammoniak oder Aminen. Obwohl diese Verfahren im großen Umfange technisch genutzt werden, ergeben sich wegen der mehrstufigen Reaktion oft nicht zufriedenstellende Ausbeuten. Auch die direkte Aminierung von Nitrobenzol mit Acetanilid in Gegenwart von Basen in DMSO ist bekannt, wobei als Hauptprodukt p-Nitrosodiphenylamin entstehen (Tetrah. Lett. 1990, 22, 3217 bis 3220). Ferner ist ein Verfahren zur Herstellung von N-aliphatisch substituierten p-Phenylendiaminen durch Umsetzung von Nitrobenzol mit aliphatischen Aminen in Gegenwart von Base und Protonen-haltigen Substanzen beschrieben (US-PS 5.252.737). Des weiteren ist ein Verfahren zur Herstellung von p-nitrosubstituierten aromatischen Amiden durch Umsetzung von Nitrobenzol mit Amiden in Gegenwart von speziellen Basen, wie Tetraalkylammoniumhydroxiden, in Gegenwart von Protonen-haltigen Stoffen beschrieben (WO 93/24447). Diese Verfahren erfordern spezielle Bedingungen und/oder liefern Ausbeuten, die nicht immer zufriedenstellend sind.

Überraschenderweise wurde nun ein allgemein anwendbares Verfahren zur direkten Amidierung von nitrosubstituierten Aromaten mit Amiden in Gegenwart von einfachen Basen und Sauerstoff gefunden. Die Umsetzungen führen in guten Ausbeuten zu den entsprechenden Amiden und gegebenenfalls durch Hydrolyse zu den Aminen.

Gegenstand der Erfindung ist ein Verfahren zur Herstellung von Verbindungen der Formel 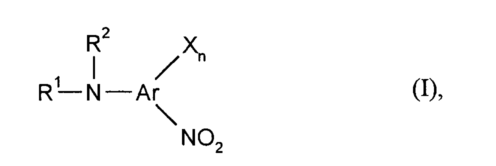
bzw. 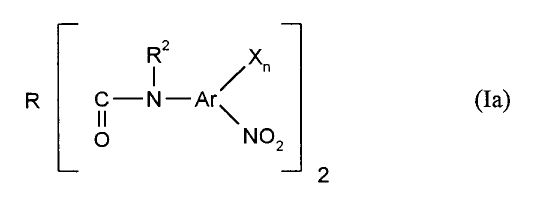
worin
- Ar: einen mono- oder polycyclischen, vorzugsweise mono- oder bicyclischen, aromatischen C₄-C₁₆-Rest, der auch 1 bis 2 Heteroatome aus der Reihe bestehend aus N, O und S enthalten kann,
- R¹: Wasserstoff, einen aliphatischen C₁-C₆-Acylrest oder einen aromatischen C₇-C₁₁-Acylrest,
- R²: Wasserstoff, C₁-C₈-Alkyl, C₂-C₈-Alkenyl, C₃-C₇-Cycloalkyl, wobei diese Substituenten 1- bis 3-fach durch Halogen, C₁-C₄-Alkyl, Amino und/oder C₁-C₄-Alkoxy substituiert sein können,
- R: eine Einfachbindung, C₁-C₈-Alkylen (vorzugsweise Tetramethylen), C₅-C₁₀-Cycloalkylen, C₆-C₁₂-Arylen (vorzugsweise Phenylen),
- X: Halogen, Cyano, C₁-C₄-Alkyl, halogeniertes C₁-C₄-Alkyl, C₁-C₄-Alkoxy, halogeniertes C₁-C₄-Alkoxy, C₁-C₄-Alkylmercapto, halogeniertes C₁-C₄-Alkylmercapto, C₁-C₄-Alkylsulfonyl oder Nitro,
- n: Null, 1, 2 oder 3, vorzugsweise Null, 1 oder 2, bedeuten,
wobei bei n >1 die Substituenten X verschieden sein können,
wonach man Nitroaromaten der Formel (II) 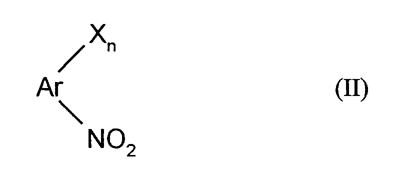
in denen
X, Ar und n die oben angegebenen Bedeutungen haben,
mit Amiden der Formel (III) 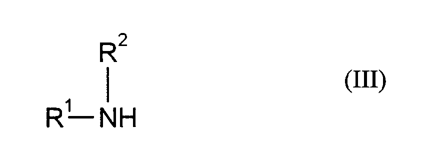
bzw. 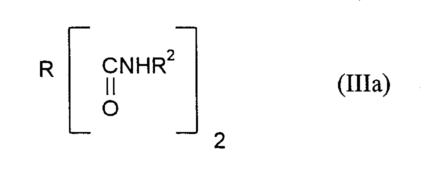
in denen
R, R¹ (R¹ ≠ H) und R² die oben angegebenen Bedeutungen haben,
in Gegenwart von Basen mit Sauerstoff in polaren Lösungsmitteln umsetzt und das resultierende Amid gegebenenfalls hydrolysiert.

Bevorzugte aromatische C₄-C₁₆-Reste umfassen beispielsweise Benzol-, Naphthalin-, Pyridin-, Chinolin- und Thiophenreste, vorzugsweise Benzol- und Naphthalinreste.

Bevorzugte aliphatische C₁-C₆- und aromatische C₇-C₁₁-Acylreste umfassen beispielsweise Formyl, Acetyl, Propionyl, Benzoyl und substituiertes Benzoyl, z.B. substituiert durch Halogen, Phenyl.

"C₁-C₈-Alkyl" und "C₁-C₄-Alkyl" umfassen lineare und verzweigte Reste wie Methyl, Ethyl, Isopropyl und n-, sek.- und tert.-Butyl.

"C₂-C₈-Alkenyl" umfaßt Vinyl und Allyl.

"C₃-C₇-Cycloalkyl" umfaßt Cyclopropyl, Cyclopentyl und Cyclohexyl.

"Halogen" steht für Brom, Iod, vorzugsweise Fluor, Chlor.

"Halogeniertes C₁-C₄-Alkyl" umfaßt z.B. Trifluormethyl und Dichlorfluormethyl.

"C₁-C₄-Alkoxy" bedeutet vorzugsweise Methoxy; "halogeniertes C₁-C₄-Alkoxy" steht vorzugsweise für Trifluormethoxy.

"C₁-C₄-Alkylmercapto" bedeutet vorzugsweise Methylmercapto; "halogeniertes C₁-C₄-Alkylmercapto" steht vorzugsweise für Trifluormethylmercapto.

"C₁-C₄-Alkylsulfonyl" steht vorzugsweise für Methylsulfonyl.

Bevorzugte Nitroaromaten II umfassen beispielsweise Nitrobenzol, m-Chlornitrobenzol, m-Nitrobenzonitril, m-Trifluormethylnitrobenzol, 3-Fluor-nitrobenzol, 3-Nitrotoluol, 3-Trifluormethoxynitrobenzol, 3-Trifluormethylthio-nitrobenzol, 3,5-Dichlornitrobenzol, 2-Nitrobenzonitril, 2-Nitrobenzoesäure, 1-Nitronaphthalin, 2-Nitronaphthalin, 2-Nitrothiophen, 3-Nitrothiophen, 2-Nitrofuran, N-alkylierte und N-arylierte 2- und 3-Nitropyrrole, 2-, 3- und 4-Nitropyridin, 4-Ethoxy-3-nitropyridin, 5-, 6- und 8-Nitrochinolin.

Bevorzugte Amide III umfassen beispielsweise Formamid, Acetamid, Propionamid, Isobuttersäureamid, N-Methyl-acetamid, N-Methyl-formamid, N-Ethyl-formamid, N-Butyl-formamid, Benzamid, 4-Methylbenzamid, 4-Methoxybenzamid, 4-Chlorbenzamid, 2-Methylbenzamid, 4-Nitrobenzamid, 4-Aminobenzamid, ferner Diamide, wie Adipinsäurediamid, Oxalsäurediamid, Terephthalsäurediamid.

Geeignete Basen sind sowohl organische wie anorganische Basen, bevorzugt werden anorganische Basen, wie z.B. Alkalihydroxide, Alkaliamide, Alkalialkoxide oder Alkalihydride. Besonders bevorzugt sind Alkalihydroxide, wie z.B. Lithiumhydroxid, Natriumhydroxid, Kaliumhydroxid, Caesiumhydroxid, Kalium-tert.-butoxid. Vorzugsweise setzt man die Basen in Form von Pulvern oder Mikrogranulat (Mikropills) ein.

Sauerstoff kann als reines Gas oder besonders bevorzugt in Gemischen mit anderen Gasen, z.B. in Form von Luft, eingesetzt werden.

Geeignete Lösungsmittel für die Herstellung der erfindungsgemäßen Verbindungen (I) bzw. (Ia) umfassen organische und anorganische Lösungsmittel. Bevorzugte organische Lösungsmittel sind polare aprotische, wie beispielsweise Dimethylsulfoxid, Dimethylformamid, N-Methylpyrrolidon, Pyridin, Dioxan, THF, Acetonitril, Sulfolan, Dimethylsulfon und deren Gemische. Ein bevorzugtes anorganisches Lösungsmittel ist beispielsweise flüssiger Ammoniak. Geringe Mengen, d.h. bis zu 10 Gew.-%, bezogen auf gesamtes Lösungsmittel, an Protonen-haltigen Lösungsmitteln, wie etwa Wasser, sind akzeptabel. Das bevorzugte Lösungsmittel ist Dimethylsulfoxid.

Die Reaktion kann innerhalb eines weiten Temperaturbereichs durchgeführt werden. Im allgemeinen arbeitet man bei Temperaturen zwischen -35 und 120°C, vorzugsweise zwischen 20 und 80°C.

Bei der Durchführung des erfindungsgemäßen Verfahrens arbeitet man im allgemeinen unter Normaldruck; es ist aber auch möglich unter erhöhtem oder vermindertem Druck zu arbeiten.

Bei der Durchführung des erfindungsgemäßen Verfahrens setzt man auf 1 Mol des Nitroaromaten (II) im allgemeinen 0,5 bis 10 Mol, vorzugsweise 0,7 bis 2 Mol an Amiden (III) bzw. (IIIa) sowie 1 bis 10 Äquivalente, vorzugsweise 2 bis 6 Äquivalente, Base ein. Sauerstoff wird vorzugsweise unverdünnt oder verdünnt im Überschuß eingeleitet.

Die Umsetzung kann so geführt werden, daß Amide oder durch nachfolgende Hydrolyse die entsprechenden Amine erhalten werden. Zusatz von Wasser beschleunigt die Hydrolyse unter alkalischen Bedingungen.

Verwendet man 3-Nitrobenzotrifluorid, Acetamid und Luft als Ausgangsstoffe und Natriumhydroxid als Base, so kann der Verlauf des Verfahren zur Herstellung von nitrosubstituierten Amiden durch das folgende Formelschema wiedergegeben werden: 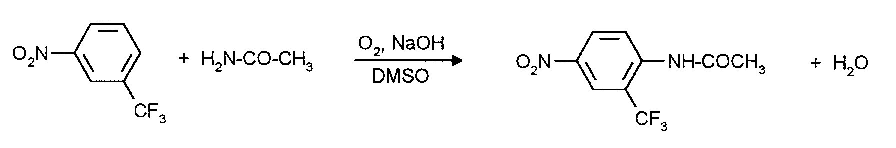
Die Ausgangsstoffe der Formeln (II) und (III) bzw. (IIIa) sind bekannt oder können nach bekannten Verfahren hergestellt werden.

Die Aufarbeitung kann nach den üblichen Methoden erfolgen. Im allgemeinen verfährt man in der Weise, daß man das Reaktionsgemisch mit Wasser stark verdünnt und das sich abscheidende Reaktionsprodukt abtrennt und isoliert, oder man verdünnt mit Wasser und extrahiert mit einem wenig mit Wasser mischbaren organischen Lösungsmittel. Aus der organischen Phase isoliert man das Produkt, nachdem diese getrocknet und eingeengt wurde.

### Beispiele

### Beispiel 1

### 4-Nitro-2-trifluormethyl-acetanilid

1,91 g (10 mmol) 3-Nitrobenzotrifluorid und 0,65 g (11 mmol) Acetamid werden mit 0,8 g (20 mmol) Natriumhydroxid in Form von Mikroperlen in 50 ml abs. DMSO vorgelegt und unter Durchleiten von einem getrockneten Luftstrom 6 h auf 50°C erhitzt. Dann wird mit Ethylacetat verdünnt und mit Wasser mehrfach ausgeschüttelt. Nach Trocknen der organischen Phase über Natriumsulfat und Abziehen des Lösemittels erhält man 2,2 g (8,5 mmol, 85 %) 4-Nitro-2-trifluormethyl-acetanilid vom Fp. 135 bis 137°C.

### Beispiel 2

### 4-Nitro-2-trifluormethyl-anilin

5 g (26 mmol) 3-Nitrobenzotrifluorid, 6 g (130 mmol) Formamid und 5,2 g (130 mmol) NaOH-Pulver werden in 100 ml DMSO unter Rühren 2 h auf 50°C unter gleichzeitigem Durchleiten von Sauerstoff erhitzt. Nach dem Abkühlen wird das Reaktionsgemisch in 600 ml Wasser und 200 g Eis gegossen. Nach 2 h Rühren wird der Niederschlag abgesaugt, mit Wasser gewaschen und getrocknet. Man erhält 3,8 g (18,5 mmol, 71 %) 4-Nitro-2-trifluormethyl-anilin vom Fp. 90-94°C.

Entsprechend den Beispielen 1 und 2 werden folgende Verbindungen hergestellt.

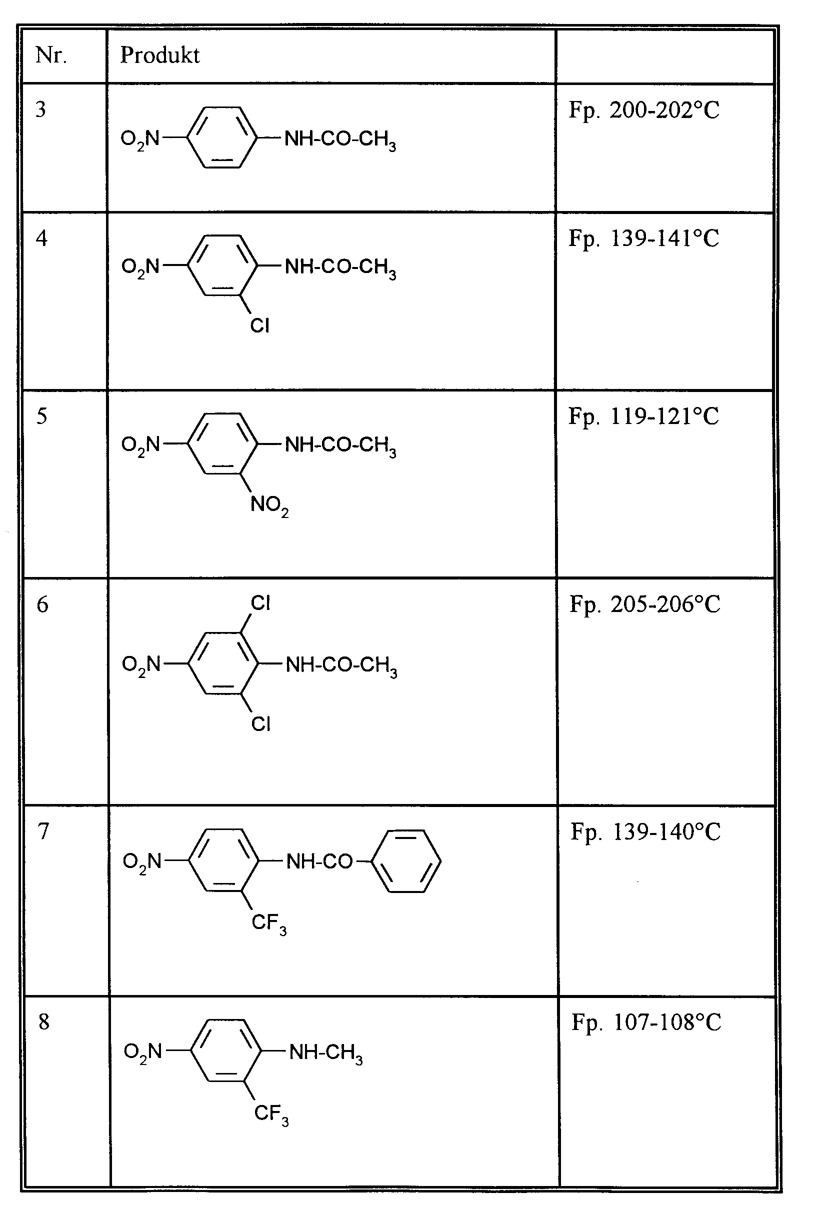

## Patentansprüche

1. Verfahren zur Herstellung von Verbindungen der Formel 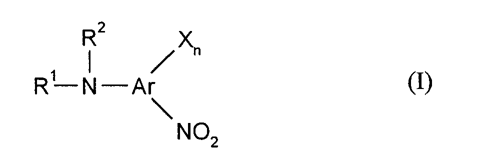 bzw. 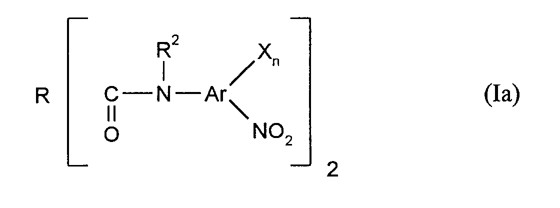 worin
Ar einen mono- oder polycyclischen aromatischen C₄-C₁₆-Rest, der auch 1 bis 2 Heteroatome aus der Reihe bestehend aus N, O und S enthalten kann,
R¹ Wasserstoff, einen aliphatischen C₁-C₆-Acylrest oder einen aromatischen C₇-C₁₁-Acylrest,
R² Wasserstoff C₁-C₈-Alkyl, C₂-C₈-Alkenyl, C₃-C₇-Cycloalkyl, wobei diese Substituenten 1- bis 3-fach durch Halogen, C₁-C₄-Alkyl, Amino und/oder C₁-C₄-Alkoxy substituiert sein können,
R eine Einfachbindung, C₁-C₈-Alkylen, C₅-C₁₀-Cycloalkylen, C₆-C₁₂-Arylen,
X Halogen, Cyano, C₁-C₄-Alkyl, halogeniertes C₁-C₄-Alkyl, C₁-C₄-Alkoxy, halogeniertes C₁-C₄-Alkoxy, C₁-C₄-Alkylmercapto, halogeniertes C₁-C₄-Alkylmercapto, C₁-C₄-Alkylsulfonyl oder Nitro,
n Null, 1, 2 oder 3 bedeuten,
wobei bei n > 1 die Substituenten X verschieden sein können,
wonach man Nitroaromaten der Formel (II) 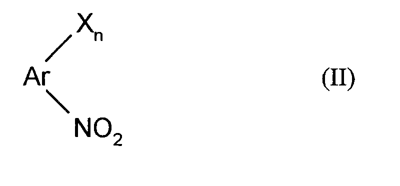 in denen
X, Ar und n die oben angegebenen Bedeutungen haben,
mit Amiden der Formel (III) 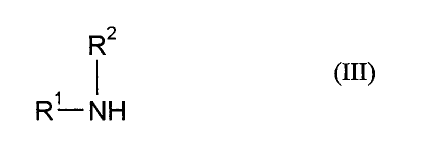 bzw. 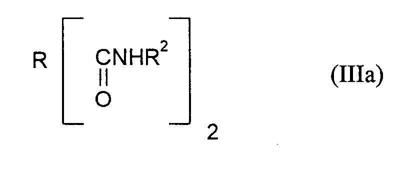 in denen
R, R¹ (R¹ ≠ H) und R² die oben angegebenen Bedeutungen haben,
in Gegenwart von Basen mit Sauerstoff in polaren Lösungsmitteln umsetzt und das resultierende Amid gegebenenfalls hydrolysiert.
